# EUROPÄISCHE PATENTSCHRIFT

(11) **EP 3 448 326 B1**
(45) Veröffentlichungstag und Bekanntmachung des Hinweises auf die Patenterteilung: **05.07.2023**
(21) Anmeldenummer: 17719268.9
(22) Anmeldetag: 26.04.2017
(51) Int. Cl.: A61F 5/01, A61F 5/14, A43B 7/20, A43B 13/22, A43B 17/00

(54) **ORTHESE MIT FUSSPLATTE**
ORTHOSIS WITH FOOT PLATE
ORTHÈSE AVEC PLAQUE DE SOUTIEN DE PIED

(30) Priorität: 29.04.2016 DE 102016108055
(43) Veröffentlichungstag der Anmeldung: 06.03.2019
(73) Patentinhaber: Ottobock SE & Co. KGaA, 37115 Duderstadt (DE)
(72) Erfinder: SIEWERT, Gordon, 37083 Göttingen (DE); TÜTTEMANN, Markus, 45731 Waltrop (DE); LÜRSSEN, Marcus, 37073 Göttingen (DE)
(74) Vertreter: Gramm, Lins & Partner Patent- und Rechtsanwälte PartGmbB
(86) Internationale Anmeldenummer: PCT/EP2017/059866
(87) Internationale Veröffentlichungsnummer: WO 2017/186761

(56) Entgegenhaltungen:
- EP-A1- 2 896 389
- DE-A1-102010 019 355
- NL-C2- 1 014 794
- US-A- 5 469 639
- US-B1- 6 301 805

## Beschreibung

Die Erfindung betrifft eine Orthese mit einer Fußplatte zur Auflage eines Fußes, mit einer davon in proximaler Richtung abstehenden Fußstrebe und einer Unterschenkelschiene zur Anlage an einen Unterschenkel, wobei die Fußplatte eine Vorderkante aufweist.

Fußplatten dienen zur Auflage eines Fußes, beispielsweise in einem Schuh als Einlegesohle oder als Auflager bei Orthesen. Sie können aus einem flexiblen Material oder einer Materialkombination bestehen, ebenso können die Fußplatten elastisch ausgebildet sein und/oder eine Stabilität oder Steifigkeit aufweisen, die es ermöglich, einen auf der Fußplatte befindlichen Fuß abzustützen und gegebenenfalls die notwendigen Kräfte und Momente zu übertragen und aufzunehmen, die bei der Benutzung der Fußplatte auftreten.

Orthesen sind orthopädietechnische Einrichtungen, die an dem Körper getragen werden, beispielsweise an einer Gliedmaße oder an dem Rumpf und über Befestigungselemente wie Gurte, Manschetten, Schuhe oder Klammern an dem jeweiligen Körperteil befestigt werden. Die Befestigungselemente können beispielsweise über Schnallen oder Klettverschlüsse verschlossen werden, um eine individuell an den Orthesenträger angepasste Festlegung an der jeweiligen Gliedmaße zu erreichen. Orthesen dienen grundsätzlich zur Unterstützung, Stabilisierung, Abstützung, Entlastung oder auch Bewegungseinschränkung der jeweiligen Gliedmaße oder Gelenke, ebenso zur Korrektur von Fehlstellungen, zur korrekten Ausrichtung oder Beibehaltung einer bestimmten Orientierung sowie zum Schutz von Gelenken und Gliedmaßen.

Im Bereich der unteren Extremitäten sind Orthesen beispielsweise als Knie-Knöchel-Fuß-Orthesen (KAFO) oder als Knöchel-Fuß-Orthesen (AFO) ausgebildet und dienen beispielsweise zur Verringerung der Auswirkungen einer Schwächung oder eines Ausfalls der Fußhebermuskulatur. Dazu kann ein federbelastetes Gelenk im Bereich des natürlichen Knöchelgelenkes vorgesehen sein, die die Fußplatte gegenüber der Unterschenkelschiene vorspannt, um in der Schwungphase eine Dorsalflexion durchzuführen, so das weiterhin ein Durchschwingen des Fußes möglich ist, ohne dass die Zehen oder Fußspitze auf dem Boden schleift. Statt eines federbelasteten Gelenkes kann auch eine Feder eine Fußplatte mit der Unterschenkelschiene verbinden.

Aus der EP 2 563 300 B1, die den nächstliegenden Stand der Technik darstellt, ist eine Orthese zur Korrektur einer Beinfehlstellung bekannt, mit einem Auflageschenkel, der einen Fuß einer Person untergreift und einen Kontakt mit einer Lauffläche, also entweder einer Sohle oder einem Fußboden herstellt. Die Orthese weist eine seitlich nach oben ragende Schienenanordnung auf, die mit einer Befestigungseinrichtung mit dem Unterschenkel der Person verbindbar ist, wobei ein bei Belastung starres Winkelstück am Übergang von dem Auflageschenkel zur Schienenanordnung ausgebildet ist. Die Schienenanordnung ist durch ein etwa in Höhe des natürlichen Knöchelgelenkes angeordnetes Drehgelenk in einen zur seitlichen Anlage an dem Fuß vorgesehenen Anlageschenkel und in eine zur seitlichen Anlage an dem Unterschenkel vorgesehene Schiene unterteilt. Die Schiene ist als federndes Element ausgebildet, über ein aus einer eingestellten Vorspannung des federnden Elementes relativ zu dem Unterschenkel resultierendes Drehmoment wird eine seitlich wirkende Korrekturkraft auf den Unterschenkel ausgeübt.

Die US 2014/0316316 A1 betrifft einen Total Contact Cast mit einem Fußpolster aus einem halbstarren Kunststoff und einem darauf aufgebrachten Schaumpolster. Von dem Fußpolster aus erstrecken sich in vertikaler Richtung zwei Unterschenkelschienen. In Querrichtung des Fußpolsters sind an seiner Unterseite mehrere Kerben jeweils über seine gesamte Breite ausgebildet.

Die US 8 574 181 B2 betrifft eine Gehhilfe bei Verletzungen im Knöchelbereich. Die Unterseite einer Schale der Gehhilfe weist eine Führungsschiene und zwei Verbindungsdurchgänge auf, die der Anbringung einer Sohle mit dazu korrespondierenden Verbindungselementen auf deren Oberseite und mit einem Laufflächenprofil auf deren Unterseite dienen.

Die EP 619 99 A1 betrifft eine Unterschenkelorthese mit einer Sohle, die an seinen gegenüberliegenden Enden jeweils über ein nach außen gekrümmtes Profil verfügt, in dem mehrere Ausnehmungen über die gesamte Breite der Sohle ausgebildet sind. Ein kürzbares Fersenpolster und zwei kürzbare Knöchelpolster werden zwischen Ferse und Orthese sowie Knöchel und Orthese eingesetzt, um die Passform zu verbessern.

Die NL 1 014 794 C2 betrifft eine Orthese mit einer Sohlenplatte und einem Unterschenkelteil, das im Fersenbereich über ein Scharnier mit der Sohlenplatte gekoppelt ist. Das Unterschenkelteil liegt im Wadenbereich an dem Unterschenkel an. Im Vorderfußbereich ist ein Scharnier in der Sohlenplatte ausgebildet. Das Scharnier ist als Filmscharnier ausgebildet.

Die US 6 301 805 B1 betrifft eine Schuheinlage für den gesamten Fuß übergewichtiger Personen. Auf der Fußsohlenunterseite sind Vorsprünge oder Noppen in Ausnehmungen im Fersenbereich und Vorfußbereich ausgebildet. Auf der Unterseite der Einlage sind Schnittlinien aufgedruckt oder eingeformt oder durch Perforation ausgebildet, so dass zur Herstellung von Einlagen für kleinere Schuhe die Innensohle durch Abreißen entlang der jeweils geeigneten Linie eine Anpassung erfolgen kann.

Die EP 2 896 389 A1 betrifft ein Orthesensystem mit einer vergleichsweise steifen Sohle, die flexible Bereiche aufweist, die sich quer über die Sohle erstrecken, um die Bewegung des Fußes während des Gehens zu steuern. Dies wird durch die Ausrichtung der flexiblen Bereiche an der Unterseite der Sohle erreicht, so dass während des Abrollens die Bewegung des Fußes in den gewünschten Richtungen unterstützt wird. Die Sohle ist über eine Manschette, die sich um den Knöchel erstreckt, an dem Unterschenkel befestigbar.

Die US 5 469 639 A betrifft eine Schuhsohle mit einem Einsatz mit abgestuften Polstereigenschaften. Der Einsatz dient zur Auflage eines Fußes und weist auf der Unterseite quer zur Längserstreckung des Einsatzes Rillen auf. Die Schuhsohle weist ein Sohlenprofil auf, das zumindest eine Quernut aufweist, deren hintere Wand schräg zur Lauffläche gerichtet ist.

Die jeweiligen Fußplatten oder Auflageschenkel werden entweder individuell angefertigt oder weisen eine Standardgröße auf, die so bemessen ist, dass die in Frage kommenden Patienten sie problemlos nutzen können. Für eine Anpassung der Fußplatte oder des Auflageschenkels an den jeweiligen Schuh oder den jeweiligen Patienten werden diese Fußplatten von einem Orthopädiemechaniker an den Schuh und/oder den Fuß angepasst, gekürzt, in der Form verändert und beschliffen. Bei Einlegesohlen für Schuhe ist es bekannt, Markierungen auf der Einlegesohle anzubringen, entlang derer eine Kürzung oder Konturänderung mit einer Schere vorgenommen werden kann, so dass die Standardformen eines Schuhs abgedeckt werden.

Aufgabe der vorliegenden Erfindung ist es, eine Orthese bereitzustellen, mit der eine erleichterte Anpassung an unterschiedliche Schuhformen und/oder einen Patienten erfolgen kann.

Erfindungsgemäß wird diese Aufgabe durch eine Orthese mit den Merkmalen des Hauptanspruches gelöst. Vorteilhafte Ausgestaltungen und Weiterbildungen der Erfindung sind in den jeweiligen Unteransprüchen, der Beschreibung sowie den Figuren offenbart.

Die Orthese mit einer Fußplatte zur Auflage eines Fußes, mit einer davon in proximaler Richtung abstehenden Fußstrebe und einer Unterschenkelschiene zur Anlage an einen Unterschenkel, wobei die Fußplatte eine Vorderkante aufweist, sieht vor, dass in der Fußplatte zumindest eine Schwächung ausgebildet ist, die sich in medial-lateraler Richtung der Fußplatte erstreckt, wobei die Schwächung zu der Vorderkante beabstandet ausgebildet ist. Durch die von der Vorderkante beabstandete Schwächung innerhalb der Fußplatte ist es möglich, entlang der Schwächung eine einfache Anpassung der Kontur oder Länge der Fußplatte vorzunehmen, so dass eine erleichterte Individualisierung der Fußplatte, z.B. als Einlegesohle und/oder der Orthese erfolgen kann. Dazu wird entlang der jeweiligen Schwächung die Fußplatte durchtrennt und das vor der Schwächung liegende Material entfernt. Die Fußplatte kann somit in Form und Länge an den jeweiligen Nutzer und/oder den Schuh angepasst werden, in dem die Fußplatte oder Orthese getragen werden soll.

Eine auf der Unterseite der Fußplatte angeordnete Schwächung weist eine von anterior nach posterior schräg nach unten gerichtete Orientierung auf, so dass die Vorderkante der Fußplatte nach Kürzung oder Konturanpassung eine schräg nach unten weisende, geradlinige oder abgerundete Orientierung aufweist, wodurch das Abrollen des Fußes entlang der Fußplatte erleichtert wird. Ist die Schwächung auf der Oberseite der Fußplatte angeordnet, verläuft die Schwächung von anterior nach posterior in einer schräg nach oben gerichteten Orientierung. Aus der anderen Blickrichtung verläuft bei einer Schwächung auf der Oberseite mit einer im Wesentlichen glatten Unterseite die Schwächung von posterior nach anterior in Richtung der Unterseite. Bei einer unterseitigen Schwächung mit einer glatten Oberseite verläuft die Schwächung von der Unterseite von posterior nach anterior in Richtung der Oberseite. Darüber hinaus wird eine abgeschrägte oder abgerundete Vorderkante einer angepassten Orthese, also auch bei einer abgerundeten Kontur mit einer Außenwölbung, die Vorderkante als weniger störend empfunden als wenn sie eine senkrecht zur Unterseite oder Oberseite verlaufende Orientierung haben würde. Bevorzugt läuft die Vorderkante der Schwächung nach der Kürzung oder Anpassung der Kontur in eine Materialdicke aus, die kleiner als 2 mm, vorzugsweise 0,5 mm beträgt.

Die Schwächung kann durchgängig ausgebildet sein, so dass entlang der in der Regel als Linie ausgebildeten Schwächung eine leichte Anpassbarkeit erfolgen kann. Ebenso ist es möglich, dass die Schwächung als unterbrochene Linie oder Perforation ausgebildet ist. Die Ausbildung der Schwächung als durchgängige Linie hat den Vorteil einer gleichmäßigen Formgebung der Außenkante hat, wodurch sich ein angenehmeres Trageverhalten ergibt und darüber hinaus keine oder nur noch geringe Nacharbeiten an der verbliebenen Fußplatte zur Glättung der neuen Vorderkante notwendig sind.

Die Schwächung kann sich über die gesamte Breite der Fußplatte erstrecken, wodurch insbesondere eine Kürzung der Fußplatte und eine Anpassung an die Wünsche oder Gegebenheiten des jeweiligen Orthesennutzers erfolgen können.

Bevorzugt folgt die Schwächung oder folgen die Schwächungen der Kontur der Vorderkante der Ausgangsform der Fußplatte oder Orthese, so dass die grundsätzliche Formgebung beibehalten wird, lediglich eine Längenanpassung der Fußplatte erfolgt. Alternativ kann jede Schwächung eine von der davor oder dahinter angeordneten Schwächung abweichende Kontur oder Form und/oder Verlauf aufweisen, um so neben der Länge auch eine andere Form in Abhängigkeit von der Länge zu erhalten.

Die Schwächung ist in einer Weiterbildung hinter oder posterior einem Metatarsalgelenk angeordnet, da durch die Materialverringerung im Bereich der Schwächung die Fußplatte als nicht störend wahrgenommen wird. Befindet sich eine Endkante vor dem Metatarsalgelenk, wird dies als wenig komfortabel empfunden, so dass ein Auslaufen der Fußplatte in oder hinter dem Metatarsalgelenk bevorzugt wird.

Mehrere Schwächungen können in gleichmäßigen Abständen hintereinander angeordnet sein, also von anteriorer Richtung in posteriorer Richtung der Fußplatte, so dass eine Vielzahl von unterschiedlichen, voreingestellten Längen und/oder Formen auswählbar sind, wodurch die Fußplatte und/oder die Orthese an viele Fußgrößen oder Fußformen oder Schuhgrößen oder Schuhformen anpassbar ist.

Die Schwächung kann als Schlitz, Ausnehmung und/oder Materialverringerung der Fußplatte ausgebildet sein. Über einen Schlitz oder eine Vielzahl von in einer Linie hintereinander angeordneten Schlitzen ist es einfach möglich, eine gewünschte Vorderkontur voreinzustellen, die durch Vertiefung des Schlitzes über die gesamte Materialstärke der Fußplatte finalisiert wird. Ebenso kann die Schwächung als Ausnehmung ausgebildet sein, beispielsweise als nachträglich durch Schleifen, Fräsen oder andere Trennverfahren eingebrachte Materialabtragungen, insbesondere an der Unterseite der Fußplatte. Auch durch Materialverringerungen, die bei der Herstellung der Fußplatte bereits vorgesehen ist, können Schwächungen in der Fußplatte eingearbeitet werden.

In einer Weiterbildung der Erfindung ist es vorgesehen, dass in der Schwächung ein Material angeordnet ist oder anordbar ist und die Schwächung ausfüllt. Das ergänzende Material kann von einem Grundmaterial der Fußplatte abweichen oder auch aus dem gleichen Material bestehen. Da die Schwächung beispielsweise als eine Nut oder eine Materialverringerung in dem Grundmaterial der Fußplatte ausgebildet ist, kann die Schwächung zur Vergleichmäßigung der Oberfläche der Fußplatte, insbesondere der Unterseite der Fußplatte, mit einem Material gefüllt oder aufgefüllt sein, das beispielsweise farblich abgesetzt ist oder auch nur teilweise mit dem Grundmaterial der Fußplatte verbunden ist. Das Material, das in die Schwächung eingebracht wird, kann lösbar eingeklebt werden. Es kann eine glatte oder zusammenhängende Oberfläche aufweisen, so dass die Fußplatte von dem Nutzer als glatt oder eben wahrgenommen wird. In dem zusätzlichen Material können Perforationslinien ausgebildet sein, entlang derer das überschüssige Material bei einer Kürzung der Fußplatte abgerissen werden kann. Alternativ ist vorgesehen, dass das auffüllende Material nach der Anpassung und Kürzung der Fußplatte in die verbliebenen Schwächungen oder in die verbliebende Schwächung eingebracht wird, beispielsweise eingeklebt, eingeklemmt oder eingeschoben, so dass nur die noch vorhandenen Schwächungen aufgefüllt werden. Ein Ausfüllen oder zumindest teilweises Auffüllen der Schwächungen ist insbesondere bei einer obenseitigen Anordnung der Schwächungen vorteilhaft. Wenn die Schwächungen dem Fuß zugewandt ausgebildet sind, ist ein Auffüllen aus Komfortgründen vorteilhaft.

Die Abweichung des zusätzlichen Materials, das von dem Grundmaterial abweichen kann, kann also in farblicher Hinsicht oder im Hinblick auf die mechanischen Eigenschaften des Materials abweichend sein. Wird entlang der Schwächung die Kontur der Fußplatte verändert, wird das abweichende Material vollständig aus der jeweiligen Schwächung entfernt, so dass die Kontur der Schwächung dann nach dem Kürzen oder Abschneiden des Materials jenseits der Schwächung die Außenkontur der Fußplatte ergibt.

Die Fußplatte ist bevorzugt im Rahmen einer Kurzfußvariante nicht über die gesamte Fußlänge ausgebildet, sondern erstreckt sich nicht über das Zehengrundgelenk hinaus nach vorn. Grundsätzlich ist jedoch auch vorgesehen, dass eine Vollfußversion der Fußplatte mit Schwächungen ausgebildet ist, um eine entsprechende Anpassung an die Schuhform und/oder Fußform zu ermöglichen.

Eine Weiterbildung der Erfindung sieht vor, dass bei einer Orthese die Fußplatte einen formstabilen Abschnitt im Bereich der Fußstrebe und einen flexiblen Abschnitt im Bereich der Vorderkante aufweist. Die Fußplatte muss nicht über die gesamte Länge formstabil sein, um die aufzunehmenden Kräfte auf den Fuß zu übertragen oder ein stabilisierendes, haltendes oder unterstützendes Moment auf den Fuß aufzubringen. Vielmehr reicht es häufig aus, wenn nur im Bereich des Knöchelgelenks bis zum Mittelfuß eine erhöhte Formstabilität vorhanden ist, so dass die Ferse und der Mittelfuß eine flächige Abstützung erfahren, während durch eine flexible Ausgestaltung im vorderen Bereich der Fußplatte ein verbessertes Abrollverhalten gewährleistet werden kann. Insbesondere für das Tragen innerhalb eines Schuhs ist ein flexibler Abschnitt im Bereich der Vorderkante vorteilhaft. Darüber hinaus wird bei einer flexiblen und gegebenenfalls elastischen Ausgestaltung eine erhöhte Nachgiebigkeit und Kompressibilität im vorderen Bereich der Fußplatte verwirklicht, was eine Erhöhung des Tragekomforts zur Folge hat.

Die Schwächung oder die Schwächungen sind bevorzugt in dem flexiblen Abschnitt ausgebildet, wodurch auch eine leichte Anpassbarkeit oder Kürzbarkeit der Fußplatte erfolgen kann, beispielsweise durch Entlangfahren eines Messers entlang der Schwächung oder durch Ausschneiden mittels einer Schere oder einem geeigneten Trennwerkzeug.

An der Fußstrebe kann eine Bodenstrebe angeformt oder befestigt sein, die sich in, auf oder unter die Fußplatte erstreckt, so dass eine zweitteilige Ausgestaltung des Fußteils mit Fußstrebe und Bodenplatte ergibt. Grundsätzlich ist es auch möglich, die Orthese einteilig auszugestalten, gegebenenfalls in einer angeformten Fußplatte oder einer angeformten oder befestigten Fußplatte, die miteinander verbunden sind.

Die Unterschenkelschiene kann über ein Gelenk um eine Gelenkachse relativ zu der Fußstrebe schwenkbar an der Fußstrebe gelagert sein. Alternativ kann statt eines Gelenkes die Unterschenkelschiene über eine Feder relativ zu der Fußplatte verlagerbar an der Fußstrebe gelagert sein, je nach gewünschtem Einsatzzweck der Orthese.

Die Fußplatte und/oder die Fußstrebe und/oder die Unterschenkelschiene können zumindest teilweise mit einer Ummantelung versehen sein, um die Trageeigenschaften zu verbessern, eine Abpolsterung zu bewirken und eine verbesserte optische Anmutung bereitzustellen.

Nachfolgend wird ein Ausführungsbeispiel der Erfindung anhand der beigefügten Figuren näher erläutert. Es zeigen:
- Figur 1 -: eine Orthese als AFO in perspektivischer Gesamtansicht;
- Figur 2 -: ein Fußteil der Orthese ohne Unterschenkelschiene in Seitenansicht;
- Figur 3 -: ein Fußteil in Untenansicht;
- Figur 4 -: ein Fußteil in Draufsicht;
- Figur 5 -: ein Fußteil in perspektivischer Schrägansicht;
- Figur 6 -: ein Fußteil in Rückansicht;
- Figuren 7 -11 -: Detailansichten einer Fußplatte mit Schwächungen an der Unterseite;
- Figuren 12 - 16 -: Detailansichten einer Fußplatte mit Schwächungen an der Oberseite; sowie
- Figur 17 -: eine schematische Darstellung der Längenanpassung einer Fußplatte.

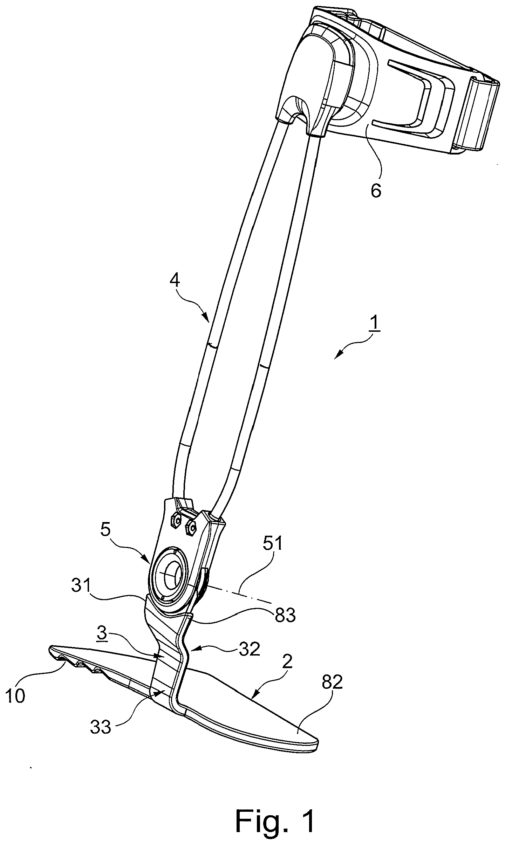

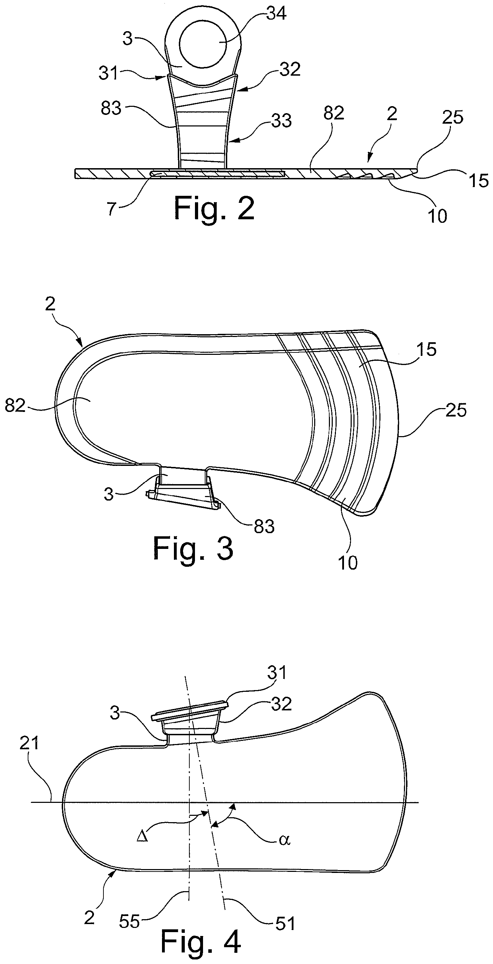

Figur 1 zeigt eine Orthese 1 in Gestalt einer Knöchel-Fuß-Orthese mit einer Fußplatte 2 zur Auflage eines nicht dargestellten Fußes, von der in proximaler Richtung eine Fußstrebe 3 absteht. Die Fußstrebe 3 ist an ihrem distalen Endbereich 33 an der Fußplatte 2 befestigt oder einstückig damit ausgebildet. Die Befestigung der Fußstrebe 3 kann durch Einlaminieren, separate Befestigungselemente wie Schrauben oder Nieten, Einkleben, Einrasten oder andere formschlüssige oder stoffschlüssige Befestigungsarten erfolgen. Ebenso ist es möglich, dass an der Fußstrebe 3 eine sich in der Ebene der Bodenplatte 2 erstreckende Bodenstrebe 7, die in der Figur 2 gezeigt ist, angeformt oder befestigt ist, die sich in, auf oder unter der Fußplatte 2 erstreckt und somit einen Winkel bildet, der eine starre, gelenklose Anbindung der Fußstrebe 3 an der Fußplatte 2 ermöglicht.

An dem proximalen Endbereich 31 der Fußstrebe 3 ist ein Gelenk 5 befestigt, das als flaches Gelenk ausgebildet ist und über das eine Unterschenkelschiene 4, an dessen proximalen Ende eine Befestigungseinrichtung 6 zur Festlegung an einem nicht dargestellten Unterschenkel angeordnet ist, schwenkbar an der Fußstrebe 3 befestigt ist. Die Befestigungseinrichtung 6 umschließt den Unterschenkel vollumfänglich und weist eine Manschette mit einem Schließgurt auf. Über zwei gebogene Streben der Unterschenkelschiene 4 ist die Neigung des oberen Anlagepunktes der Unterschenkelschiene 4 relativ zu dem Unterschenkel bzw. relativ zu der Orientierung der Fußstrebe 3 einstellbar. Neben der dargestellten Orthese zur Korrektur einer Beinfehlstellung kann die Fußstrebe 3 gegenüber der Unterschenkelschiene 4 federnd vorgespannt sein, beispielsweise über eine in dem Gelenk 5 angeordnete Feder, um eine Fußheberorthese bereitzustellen.

Die Unterschenkelschiene 4 ist über das Gelenk 5 relativ zu der Fußstrebe 3 um eine Gelenkachse 51 verschwenkbar, eine gelenkige Verschwenkung um eine andere Achse ist nicht vorgesehen.

Die Fußplatte 2 weist eine Ummantelung 82 auf, die aus einem flexiblen, gegebenenfalls elastischen Werkstoff bestehen kann. An der Unterseite der Fußplatte 2 sind im dargestellten Ausführungsbeispiel drei hintereinander angeordnete Schwächungen 10 ausgebildet, entlang der die Fußplatte 2 einfach kürzbar ist. Die Schwächungen 10 weisen eine Sägezahnform auf, so dass sich nach dem Abtrennen des vor der Schwächung 10 liegenden Materials eine nach unten geneigte oder abgerundete Vorderkante 25 ergibt, die ein Abrollen erleichtert und weniger störend empfunden wird. Die Fußplatte 2 wird sinnvollerweise entweder an der schmalsten Stelle der Schwächung 10 oder in Fortführung der geneigten oder abgerundeten Vorderkantenform gekürzt oder in Form gebracht.

Auch an der Fußstrebe 3 ist eine Ummantelung 83 angeordnet, die sich bis zum proximalen Ende 31 der Fußstrebe 3 erstreckt und lediglich die Gelenkeinrichtung 5 und den angrenzenden Bereich des proximalen Endbereiches 31 freilässt, so dass eine freie Verschwenkung der Unterschenkelschiene relativ zu der Fußstrebe 3 nicht behindert wird. Die Ummantelung 82, 83 verbessert den Tragekomfort, da die gegebenenfalls scharfkantigen Werkstoffe der Fußstrebe 3 und/oder der Fußplatte 2 dadurch nicht in direkten Kontakt mit dem Körper des Orthesennutzers kommen.

An der Fußstrebe 3 ist im proximalen Endbereich 31 ein Absatz 32 ausgebildet, der von der Fußplatte 2 weg weist, so dass der proximale Endbereich 31 der Fußstrebe 3 versetzt und weiter entfernt von der Fußplatte 2 als der distale Bereich 33 der Fußstrebe 3 ist. Über den Absatz 32 wird der Bereich, in dem oder an dem das Gelenk 5 angeordnet ist, von der Fußplatte 2 entfernt, so dass bei einer medialen Anordnung der Fußstrebe 3 der proximale Endbereich 31 medial versetzt zu der Fußplatte 2 befindlich ist. Bei einer lateralen Anordnung der Fußstrebe 3 erfolgt über den Absatz 32 ein Versatz in lateraler Richtung, also von dem Fuß weg. Durch den Absatz 32 und die dadurch vergrößerte Entfernung des proximalen Endbereiches 31 der Fußstrebe 3 im Vergleich zu einem distalen Endbereich 33 der Fußstrebe, beispielsweise im Bereich des Überganges von der Fußplatte 2 zur Fußstrebe 3, ist es möglich, dass die Fußstrebe 3 sehr nah an der knöchernen Struktur des Fußes entlanggeführt werden kann, ohne dass das Gelenk 5 im Bereich des Knöchels an dem knöchernen Knöchelvorsprung scheuert oder unangenehm anliegt.

Die Fußstrebe 3 weist in dem Ausführungsbeispiel einen distalen Endbereich 33 auf, der sich im Wesentlichen geradlinig von der Fußplatte 2 weg erstreckt, der distale Endbereich 33 und der proximale Endbereich 31 sind durch den Absatz 32 hinsichtlich einer Sagittalebene versetzt zueinander. Zumindest der distale Endbereich 33 der Fußstrebe 3 ist als Flachmaterial ausgebildet, im dargestellten Ausführungsbeispiel ist die gesamte Fußstrebe 3 mit distalem Endbereich 33, Absatz 32 und proximalem Endbereich 31 aus einem Flachmaterial, insbesondere einem Metall ausgebildet.

Figur 2 zeigt das Fußteil der Orthese ohne die Unterschenkenschiene 4 und ohne die Gelenkeinrichtung mit der Fußplatte 2, die eine im Wesentlichen ebene Oberseite und eine Unterseite mit Schwächungen 10 im vorderen Bereich aufweist. Die Schwächungen 10 sind sägezahnförmig ausgebildet und bilden Sollschnittstellen, entlang derer eine Kürzung der Fußplatte 2 und damit eine Anpassung an verschiedene Schuhformen oder Fußformen erfolgen kann. Die Schwächungen 10 weisen eine Schräge oder vordere Kontur 15 auf, die von vorn nach hinten in einer schräg nach unten geneigten Richtung verlaufen, so dass ein Abrollen erleichtert wird. Sofern die Schräge in einer Spitze ausläuft, ist an der Vorderkante der Fußplatte 2 kein Absatz ausgebildet. Nach Möglichkeit wird ein Absatz von kleiner 2 mm, vorzugsweise kleiner 0,5 mm angestrebt, um auch im empfindlichen Fußbereich keine störenden Einflüsse beim Abrollen zu erzeugen. In der seitlichen Schnittdarstellung gemäß Figur 2 ist die Bodenstrebe 7 zu erkennen, die im dargestellten Ausführungsbeispiel mit der Fußstrebe 3 ausgebildet ist und ein gelenkloses Winkelstück darstellt. Die Bodenstrebe 7 ist mit einer Ummantelung 82 versehen, so dass aus der Bodenstrebe 7 und der Ummantelung 82 die Fußplatte 2 gebildet wird. Die Ummantelung 82 erstreckt sich auch über einen Teil der Fußstrebe 3 und ist dort mit dem Bezugszeichen 83 versehen.

Der distale Abschnitt 33 der Fußstrebe 3 erstreckt sich im Wesentlichen senkrecht nach oben von der Fußplatte 2, daran schließt sich ein Absatz 32 an, in dem das Material der Fußstrebe 3 zunächst nach außen und dann nach oben gebogen ist, so dass im proximalen Endbereich 31 eine Gelenkeinrichtung in einer Aufnahme 34, die im dargestellten Ausführungsbeispiel als runde Ausnehmung ausgebildet ist, oder das Lager für die schwenkbare Befestigung der Unterschenkelschiene 4 angeordnet werden kann.

Figur 3 zeigt das Fußteil gemäß Figur 2 in Untenansicht, die Schwächungen 10, im Ausführungsbeispiel drei Schwächungen 10, verlaufen korrespondierend zu der Vorderkante 25 der Fußplatte 2 in gleichmäßigen Abständen hintereinander und über die gesamte Breite der Fußplatte 2. Die Fußstrebe 3 ist aus der Fußplatte 2 herausgebogen.

Figur 4 zeigt eine Draufsicht auf das Fußteil mit der Fußplatte 2 und der senkrecht aus der Blattebene herausstehenden Fußstrebe 3. Die Fußstrebe 3 ist, wie bei allen anderen dargestellten Ausführungsformen, medial angeordnet, ungefähr im Bereich des knöchernen Knöchelvorsprunges des Schienbeins, und erstreckt sich senkrecht nach oben. Es ist ein minimaler Biegeradius vorgesehen, mit dem die Fußstrebe 3 über den distalen Bereich 33 mit der Fußplatte 2 verbunden ist, so dass der distale Bereich 33 der Fußstreber 3 möglichst nah an der knöchernen Struktur des Fußes anliegen kann.

Der Absatz 32 ist asymmetrisch ausgebildet, das vordere Ende des Absatzes 32 ist weiter nach medial gebogen als das hintere Ende, so dass sich in Draufsicht eine Verschwenkung oder eine winklige Orientierung des distalen Endbereiches 31 relativ zu der angedeuteten natürlichen Knöchelgelenksachse 55 ergibt. Die Gelenkachse 51 des Gelenks 5 ist in einem Winkel Δ zu der natürlichen Gelenkachse orientiert ausgerichtet und in einer horizontalen Ebene nach vorn, also in anteriorer Richtung verkippt. Der Winkel Δ zwischen der natürlichen Gelenksache 55 und der Gelenkachse 51 der Gelenkeinrichtung 5 beträgt zwischen 5° und 20°, bevorzugt zwischen 7° und 15°, insbesondere 12°, um eine Außenrotation beim Gehen oder Stehen zu kompensieren. Dadurch ist die Gelenkachse 51 in der Horizontalebene geneigt zu der Mittellinie 21 der Fußplatte 2, die im Wesentlichen der Mittellinie eines Fußes entspricht und von dem mittleren Fersenbereich senkrecht zu der Knöchelgelenkachse 55 in anterior-posterior-Richtung verläuft, orientiert. Der Winkel α der Gelenkachse 51 zur Fußmittellinie 21 beträgt somit zwischen 85° und 70° und ist dabei nach anterior gerichtet, also nach vorn verdreht orientiert.

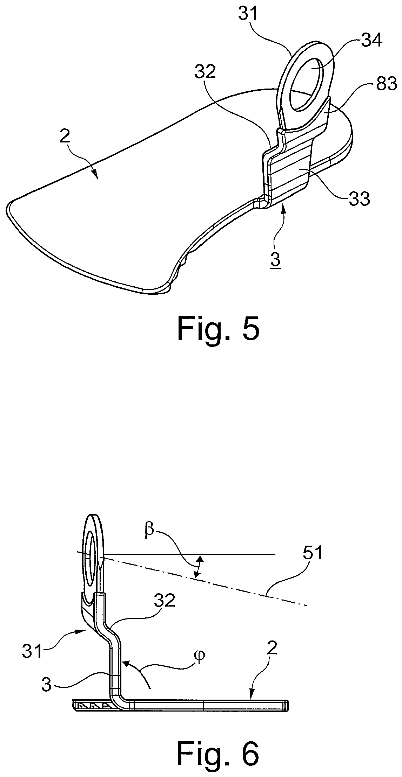

Figur 5 zeigt das Fußteil in einer perspektivischen Schrägdraufsicht, in der zu erkennen ist, dass die Fußstrebe 3 mit dem distalen Abschnitt 33 im Wesentlichen senkrecht von der Fußplatte 2 absteht. Der Absatz 32 führt dazu, dass der proximale Endbereich 31 mit der Ausnehmung 34 als Befestigungsaufnahme für die Gelenkeinrichtung 5 ebenfalls senkrecht nach oben orientiert ist, allerdings in Längserstreckung der Fußstrebe 3 nach vorn oder in anterior-Richtung verdreht.

Figur 6 zeigt das Fußteil in Hintenansicht, der Winkel ϕ zwischen der Fußplatte 2 und dem distalen Bereich der Fußstrebe 3 beträgt im dargestellten Ausführungsbeispiel 90°, was bei einer medialen Anordnung der Fußstrebe 3 zu einer guten Anlage an dem Fußknochen führt. Eine individuelle Anpassung kann durch eine plastische Verformung der Fußstrebe 3 relativ zu der Fußplatte 2 erfolgen, so dass die Fußstrebe 3 möglichst nahe an dem Fuß anliegt. Der distale Endbereich 31 der Fußstrebe 3 kann neben einer Verdrehung um die Längserstreckung in anteriorer Richtung ebenfalls eine Neigung aufweisen, so dass sich ein Winkel β zwischen der Gelenkachse 51 und der dargestellten Horizontalen ergibt. Dadurch wird es ermöglicht, dass die Gelenkachse 51 des nicht dargestellten Gelenkes individuell anpassbar ist. Die Gelenkachse 51 ist bevorzugt so positioniert, dass sie mit der natürlichen Knöchelgelenkachse zusammenfällt. Eine Höheneinstellung kann sich durch eine Winkeleinstellung der Fußstrebe 3, durch eine Änderung des Absatzes 32, durch Distanzelemente oder durch Einsätze oder Aufsätze bei der Befestigung der Unterschenkelschiene 4 ergeben.

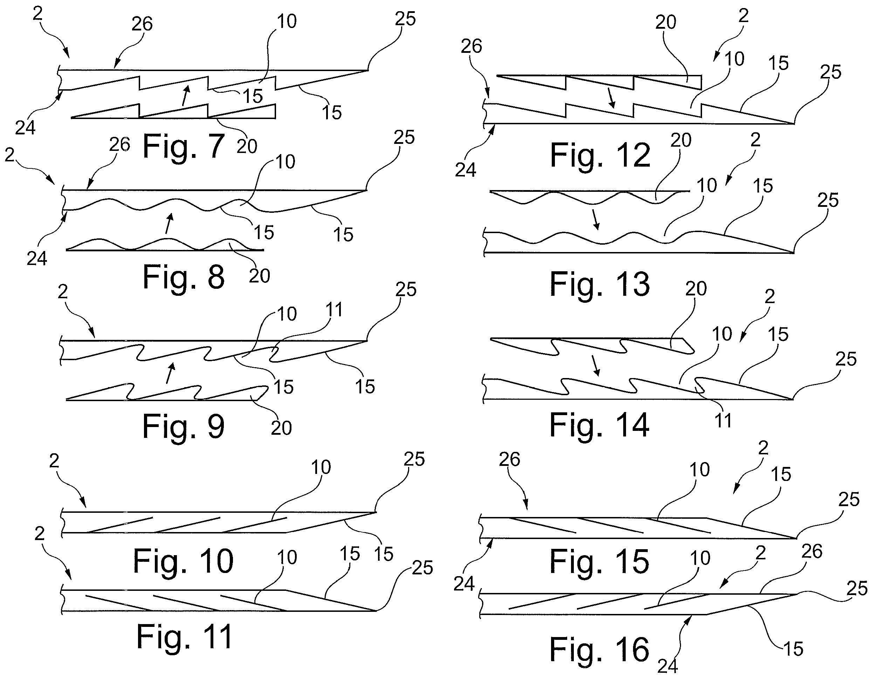

Figur 7 zeigt in einer Detailansicht ein vorderes, anteriores Ende einer Fußplatte 2 mit einer Vorderkante 25, einer Oberseite 26 und einer Unterseite 24. Von der Oberseite 26 der Vorderkante 25 erstreckt sich die vordere Kontur 15 schräg in Richtung auf die Unterseite 24 nach hinten, so dass sich die Fußplatte 2 in vorderer, anteriorer Richtung verjüngt und ausläuft. Dadurch wird ein Abrollen der Fußplatte 2 erleichtert und der Tragekomfort erhöht. An die erste Kontur 15, die sich an der Vorderkante 25 anschließt, schließen sich im dargestellten Ausführungsbeispiel drei Schwächungen 10 an, die sägezahnförmig ausgebildet sind und eine korrespondierende Kontur 15 aufweisen, also eine von vorn nach hinten schräg nach unten zur Unterseite 24 hin verlaufende Orientierung aufweisen. Zusätzlich zu der Fußplatte 2 ist ein Material 20 vorbereitet, das als Materialstreifen ausgebildet ist und eine Kontur auf der Oberseite aufweist, die der Kontur der Fußplatte 2 auf der Unterseite 24 im Bereich der Schwächungen 10 entspricht. Im dargestellten Ausführungsbeispiel der Figur 7 weist die Oberseite des ergänzenden Materialstreifens ebenfalls eine sägezahnförmige Kontur auf, mit einer jeweils geradlinigen Schräge von der Vorderkante, also anterior, nach schräg unten zur glatten Unterseite, also in posteriorer Richtung. Korrespondierend zu den drei Schwächungen 10 in der Fußplatte 2 sind drei sägezahnförmige Erhöhungen aus dem zusätzlichen Material 20 ausgebildet und können zur Ergänzung der Kontur der Fußplatte 2 an deren Unterseite 24 angeordnet oder befestigt, insbesondere eingeklebt sein. Das zusätzliche Material 20 füllt die Schwächungen 10 im dargestellten Ausführungsbeispiel vollständig aus, grundsätzlich ist es auch möglich, dass die Schwächungen 10 nur teilweise ausgefüllt werden. Das Material 20 kann von dem Material der Fußplatte 2 abweichend ausgebildet sein. Es können unterschiedliche Farbgebungen vorhanden sein, ebenfalls ist es möglich, dass das Material 20 aus demselben Material wie die Fußplatte 2 besteht. Im dargestellten Ausführungsbeispiel ergänzt das Material 20 die Fußplatte 2 so, dass die Unterseite 24 nahezu durchgehend eine ebene Fläche ausbildet. Das Material 20 erstreckt sich über die gesamte Breite der Fußplatte 2 und über die Länge der Schwächungen 10 in der Fußplatte 2, grundsätzlich ist es auch möglich, das Material 20 nur bereichsweise in die Schwächungen 10 einzusetzen und somit nicht die komplette Unterseite 24 der Fußplatte 2 auszufüllen.

Figur 8 zeigt eine Variante der Erfindung, bei der statt einer geradlinigen Schräge als Kontur 15 eine wellenförmige Kontur 15 von der Vorderkante 25 in Richtung auf das hintere Ende der Fußplatte 2 verläuft. Auch hier sind drei hintereinander angeordnete, zueinander gleich beabstandete Schwächungen 10 vorgesehen, in die das ergänzende Material 20 eingebracht werden kann. Die Oberseite des ergänzenden Materials 20 weist eine den Schwächungen 10 korrespondierende Formgebung auf und kann in den Schwächungen 10 eingeklebt, eingeklemmt oder auf andere Art und Weise darin gehalten sein. Sofern das ergänzende Material 20 andere mechanische Eigenschaften aufweist, kann über das Zusatzmaterial 20 eine Modifikation der Eigenschaften, insbesondere der Abrolleigenschaften und des Komforts, der Fußplatte 2 erreicht werden.

Figur 9 zeigt eine weitere Variante der Erfindung mit auf der Unterseite der Fußplatte 2 ausgebildeten Schwächungen 10, bei der diese nach der gewölbten, schräg nach unten und hinten gerichteten Kontur 15 einen Hinterschnitt 11 ausbilden. Es ist somit eine pfeilartige Kontur 15 mit hintereinander angeordneten Hinterschnitten 11 auf der Unterseite 24 der Fußplatte 2 ausgebildet. Durch den Hinterschnitt 11 ist es möglich, eine formschlüssige Verriegelung gegen ein Herausfallen eines zusätzlichen Materialstreifens 20 aus den Schwächungen in Richtung auf die Unterseite 24 bereitzustellen. Durch eine elastische Ausgestaltung entweder der Fußplatte 2 und/oder der ergänzenden Materialstreifen 20 kann eine klemmende und klebende sowie formschlüssige Kopplung innerhalb der Schwächungen 10 erreicht werden. Auch hier ergänzt der zusätzliche Materialstreifen 20 die Fußplatte 2 im Bereich der Schwächungen 10 zu einer durchgehenden Oberfläche mit einer im Wesentlichen glatten Unterseite 24.

Eine Variante der Erfindung ist in der Figur 10 dargestellt, bei der ebenfalls eine von der Vorderkante schräg nach unten verlaufende Kontur 15 ausgebildet ist. Statt der Schwächungen 10 in Gestalt von entfernten Materialien ist in der Variante der Figur 10 vorgesehen, dass die Schwächungen 10 durch Schlitze ausgebildet sind, die im Wesentlichen parallel zu der ursprünglich vorderen Kontur 15, die sich von der Vorderkante 25 schräg nach unten in Richtung der Unterseite 24 erstreckt, verlaufen. Die Ausgestaltung der Schwächungen 10 als Schlitze hat den Vorteil, dass im angelegten oder eingelegten Zustand eine im Wesentlichen ebene, kontinuierliche und glatte Unterseite 24 vorhanden ist. Bei einer Abrollbewegung geben die Schlitze 10 als Schwächungen nach und erleichtern ein Abrollen, wohingegen weiterhin eine ausreichende Abstützung gegenüber Kräften erfolgt, die von der Oberseite aus einwirken, so dass ein aufgesetzter Fuß von der Fußplatte 2 weiterhin unterstützt werden kann.

Eine weitere Variante der Erfindung mit Schwächungen 10 auf der Unterseite 24 ist in der Figur 11 dargestellt, bei der jedoch der Verlauf der vorderen Kontur 15 nicht von anterior nach posterior schräg nach unten verläuft, sondern schräg nach oben. Korrespondierend sind die Schwächungen 10 im Wesentlichen parallel zu der vorderen Kontur 15 von vorne nach hinten schräg nach oben verlaufend ausgebildet.

Eine Variante der Erfindung ist in den Figuren 12 bis 16 dargestellt, die im Wesentlichen den Ausführungsformen gemäß der Figuren 7 bis 11 entsprechen, wobei jedoch die Fußplatte 2 eine geschlossene, im Wesentlichen glattflächige Unterseite 24 aufweist und im anterioren, vorderen Bereich auf der Oberseite 26 die Schwächungen 10 ausgebildet sind. Auch hier können die Schwächungen 10 durch zusätzliche Materialstreifen 20 zumindest teilweise aufgefüllt werden, um eine im Bereich der Schwächungen 10 ebenfalls glatte, möglichst geschlossene Oberfläche bereitzustellen. Figur 12 entspricht in der Formgebung der Figur 7, mit dem Unterschied, dass das zusätzliche Material 20 von oben auf die Oberseite 26 der Fußplatte 2 aufgesetzt oder aufgelegt und in den Schwächungen 10 oder dem Schwächungsbereich fixiert wird. Die Kontur 15 verläuft von anterior nach posterior in Richtung von der Unterseite nach der Oberseite ansteigend, also in einer Schräge nach oben. Die Kontur der Schwächungen 10 entspricht der Kontur 15 der Fußplatte 2, wie sie sich von der Vorderkante 25 in posteriorer Richtung erstreckt.

Figur 13 zeigt eine wellenförmige Ausgestaltung der Schwächungen 10 mit drei hintereinander angeordneten Schwächungen und einer schrägen, gewölbten Kontur 15, die sich von der Vorderkante 25 in Richtung auf das posteriore Ende der Fußplatte 2 erstreckt. Das zusätzliche Material 20 füllt die Schwächungen 10 vollständig aus.

Der Hinterschnitt 11 in der Formgebung der Schwächungen 10 verhindert ein Rutschen in Richtung auf die Vorderkante 25, wenn ein zusätzliches, ergänzendes Material 20 in die Schwächungen 10 eingelegt wird. Darüber hinaus wird wirksam eine Entfernung senkrecht zu der Unterseite 24 durch eine formschlüssige Sperre verhindert.

Figur 15 zeigt die Ausgestaltung der Schwächungen 10 in Form von Schlitzen, die sich von der Oberseite 26 von posterior nach anterior schräg in Richtung auf die Unterseite 24 erstrecken und somit im Wesentlichen parallel zu der vorderen Kontur 15 verlaufen, die von der Vorderkante 25 in posteriorer Richtung von der Unterseite 24 in Richtung auf die Oberseite 26 schräg verläuft. Die Schlitze 10 sind im dargestellten Ausführungsbeispiel nicht durchgängig ausgebildet, das heißt, dass sie nicht bis zur Unterseite reichen. Gleiches gilt auch für die Schlitze oder Schwächungen gemäß der voranstehend beschriebenen Figuren 7 bis 14. Grundsätzlich ist es möglich, dass bereichsweise die Schwächungen 10 die Fußplatte 2 vollständig durchdringen, so dass eine Art Perforation in der Fußplatte 2 ausgebildet wird. Die Oberseite 26 oder die Unterseite 24 sind dann nicht vollständig geschlossen ausgebildet, sondern weisen Ausnehmungen, Schlitze, Löcher oder ähnliches auf.

Figur 16 zeigt eine Variante der Erfindung, bei der von der Vorderkante 25 die Kontur 15 von der Oberseite 26 schräg nach hinten und unten verläuft, während die Schwächungen 10 in Gestalt von Schlitzen dazu korrespondierend von der Oberseite 26 schräg in Richtung auf die Unterseite nach hinten geneigt verlaufen. Die Schlitze 10 sind im Wesentlichen parallel zu der Kontur 15 von der Vorderkante 25 ausgehend orientiert.

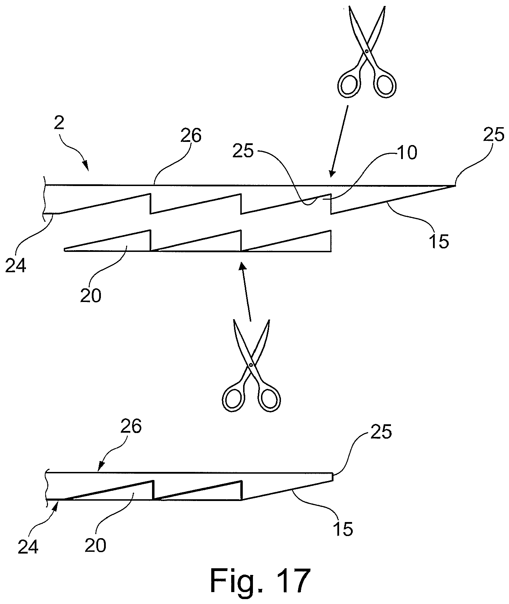

In der Figur 17 ist der Vorgang einer Anpassung der Fußplatte 2 an die Nutzerwünsche gezeigt. Zunächst wird in einem Bereich einer Schwächung 10, und zwar im vorderen Bereich der Schwächung, bei der die Kontur 15 der Schwächung maximal an der Oberseite 26 angenähert ist, mittels einer Schere der anteriore, vordere Bereich abgeschnitten. Korrespondierend dazu wird der erste, sägezahnartige Bereich des ergänzten Materials 20 im Bereich der geringsten Materialstärke mit einer Schere abgeschnitten. Dadurch wird die Anzahl der Vorsprünge des ergänzenden Materials 20 an die Anzahl der noch verbliebenen Schwächungen 10 in der Fußplatte 2 angepasst. Im vorliegenden Ausführungsbeispiel sind nach dem Kürzen des ergänzenden Materials 20 nur noch zwei sägezahnartige Erhebungen vorhanden, die in die zwei verbliebenen Schwächungen 10 innerhalb der Fußplatte 2 an der Unterseite eingesetzt werden können.

Nach dem Einsetzen und gegebenenfalls Verkleben oder Verbinden oder anderweitigem Fixieren des zusätzlichen Materials 20 in die Fußplatte 2 ergibt sich die in der unteren Darstellung gezeigte Ausgestaltung der fertigen Fußplatte 2 mit einer neuen, gekürzten und gegebenenfalls abgerundeten oder mit einer geringen Breite versehenen Vorderkante 25, einer schräg nach unten verlaufenden Kontur 25 und einer glatten Unterseite 24, da die verbliebenen Schwächungen 10 der ursprünglichen Fußplatte 2 mit dem ergänzenden Material 20 ausgefüllt sind. Somit ist eine Fußplatte 2 mit einer geschlossenen, ebenen Oberseite 26 sowie einer ebenen Unterseite 24 vorhanden.

## Patentansprüche

1. Orthese mit einer Fußplatte (2) zur Auflage eines Fußes, wobei die Fußplatte (2) eine Vorderkante (25) sowie eine, von der Fußplatte (2) in proximaler Richtung abstehende Fußstrebe (3) und eine Unterschenkelschiene (4) zur Anlage an einen Unterschenkel aufweist,
**dadurch gekennzeichnet, dass** in der Fußplatte (2) zumindest eine von der Vorderkante (25) beabstandete Schwächung (10) ausgebildet ist, die sich in medial-lateral-Richtung der Fußplatte (2) erstreckt, wobei eine untenseitige Schwächung (10) eine von anterior nach posterior schräg nach unten gerichtete Orientierung oder abgerundete Kontur (15) und eine obenseitige Schwächung (10) eine von anterior nach posterior schräg nach oben gerichtete Orientierung oder abgerundete Kontur (15) aufweist.

2. Orthese nach Anspruch 1, **dadurch gekennzeichnet, dass** die Fußplatte (2) einen formstabilen Abschnitt im Bereich der Fußstrebe (3) und einen flexiblen Abschnitt im Bereich der Vorderkante (25) aufweist.

3. Orthese nach Anspruch 2, **dadurch gekennzeichnet, dass** die Schwächung (10) oder die Schwächungen (10) in dem flexiblen Abschnitt ausgebildet sind.

4. Orthese nach einem der voranstehenden Ansprüche, **dadurch gekennzeichnet, dass** an der Fußstrebe (3) eine Bodenstrebe (7) angeformt oder befestigt ist, die sich in, auf oder unter die Fußplatte (2) erstreckt.

5. Orthese nach einem der voranstehenden Ansprüche, **dadurch gekennzeichnet, dass** die Unterschenkelschiene (4) über ein Gelenk (5) um eine Gelenkachse (51) relativ zu der Fußstrebe (3) schwenkbar an der Fußstrebe (3) gelagert ist.

6. Orthese nach einem der voranstehenden Ansprüche, **dadurch gekennzeichnet, dass** die Fußstrebe (3) und die Fußplatte (2) einstückig ausgebildet sind.

7. Orthese nach einem der voranstehenden Ansprüche, **dadurch gekennzeichnet, dass** die Fußplatte (2) und/oder die Fußstrebe (3) zumindest teilweise mit einer Ummantelung (82, 83) versehen sind.

8. Orthese nach einem der voranstehenden Ansprüche, **dadurch gekennzeichnet, dass** die Schwächung (10) durchgängig, als unterbrochene Linie oder Perforation ausgebildet ist.

9. Orthese nach einem der voranstehenden Ansprüche, **dadurch gekennzeichnet, dass** sich die Schwächung (10) über die gesamte Breite der Fußplatte (2) erstreckt.

10. Orthese nach einem der voranstehenden Ansprüche, **dadurch gekennzeichnet, dass** die Schwächung (10) der Kontur der Vorderkante (25) folgt.

11. Orthese nach einem der voranstehenden Ansprüche, **dadurch gekennzeichnet, dass** die Schwächung (10) posterior einem Metatarsalgelenk angeordnet ist.

12. Orthese nach einem der voranstehenden Ansprüche, **dadurch gekennzeichnet, dass** mehrere Schwächungen (10) in gleichmäßigen Abständen hintereinander angeordnet sind.

13. Orthese nach einem der voranstehenden Ansprüche, **dadurch gekennzeichnet, dass** die Schwächung (10) als Schlitz, Ausnehmung und/oder Materialverringerung ausgebildet ist.

14. Orthese nach einem der voranstehenden Ansprüche, **dadurch gekennzeichnet, dass** in der Schwächung (10) ein Material (20) angeordnet oder anordbar ist, das die Schwächung (10) ausfüllt.

15. Orthese nach einem der voranstehenden Ansprüche, **dadurch gekennzeichnet, dass** die Fußplatte (2) sich nicht über das Zehengrundgelenk hinaus nach vorn erstreckt.

## Claims

1. Orthotic with a foot plate (2) for supporting a foot wherein the foot plate (2) has a front edge (25) and comprises a foot support (3) in the proximal direction of the same and a lower leg support bar (4) to be applied to a lower leg, **characterized in that** the foot plate (2) has at least one weakening (10) which is spaced apart from the front edge (25) and extends in the medial-lateral direction of the foot plate (2),wherein a weakening (10) on the underside has an orientation or rounded contour (15) directed obliquely downward from anterior to posterior and a weakening (10) on the upper side has an orientation or rounded contour (15) directed obliquely upwards from anterior to posterior.

2. Orthotic according to claim 1, **characterized in that** the foot plate (2) comprises a dimensionally stable section in the area of the foot support (3) and a flexible section in the area of the front edge (25).

3. Orthotic according to claim 2, **characterized in that** the weakening (10) or the weakenings (10) are formed in the flexible section.

4. Orthotic according to any of the preceding claims, **characterized in that** a floor support (7) is formed or attached to the foot support (3) that extends in, on, or under the foot plate (2).

5. Orthotic according to any of the preceding claims, **characterized in that** the lower leg support bar (4) is positioned rotationally on the foot support (3) above a joint (5) around a joint axis (51) relative to the foot support (3).

6. Orthotic according to any of the preceding claims, **characterized in that** the foot support (3) and the foot plate (2) are made from one piece.

7. Orthotic according to any of the preceding claims, **characterized in that** the foot plate (2) and/or the foot support (3) are at least partially provided with sheathing (82, 83).

8. Orthotic according to any of the preceding claims, **characterized in that** the weakening (10) is formed continuously as a broken line or perforation.

9. Orthotic according to any of the preceding claims, **characterized in that** the weakening (10) extends across the entire width of the foot plate (2).

10. Orthotic according to any of the preceding claims, **characterized in that** the weakening (10) follows the contour of the front edge (25).

11. Orthotic according to any of the preceding claims, **characterized in that** the weakening (10) is arranged posterior to a metatarsal joint.

12. Orthotic according to any of the preceding claims, **characterized in that** a plurality of weakenings (10) are consecutively arranged at equal distances.

13. Orthotic according to any of the preceding claims, **characterized in that** the weakening (10) is formed as a slit, recess and/or reduction in material.

14. Orthotic according to any of the preceding claims, **characterized in that** material (20) is arranged or is arrangeable in the weakening (10) that fills the weakening (10).

15. Orthotic according to any of the preceding claims, **characterized in that** the foot plate (2) does not extend past the metatarsal joint.

## Revendications

1. Orthèse comprenant une plaque repose-pied (2) pour supporter un pied, la plaque repose-pied (2) présentant un bord avant (25) ainsi qu'une entretoise de soutien de pied (3), dépassant de la plaque repose-pied (2) dans la direction proximale, et une éclisse de bas de jambe (4) pour l'appui contre un bas de jambe,
**caractérisée en ce qu'**au moins un affaiblissement (10) espacé du bord avant (25) est formé dans la plaque repose-pied (2), lequel s'étend dans la direction médiale-latérale de la plaque repose-pied (2), un affaiblissement (10) côté inférieur présentant une orientation ou un contour arrondi (15) dirigé(e) en oblique vers le bas dans la direction antéro-postérieure, et un affaiblissement (10) côté supérieur présentant une orientation ou un contour arrondi (15) dirigé(e) en oblique vers le haut dans la direction antéro-postérieure.

2. Orthèse selon la revendication 1,
**caractérisée en ce que** la plaque repose-pied (2) présente une portion solide en forme dans la zone de l'entretoise de soutien de pied (3) et une portion flexible dans la zone du bord avant (25).

3. Orthèse selon la revendication 2,
**caractérisée en ce que** l'affaiblissement (10) ou les affaiblissements (10) sont formés dans la portion flexible.

4. Orthèse selon l'une des revendications précédentes,
**caractérisée en ce qu'**une entretoise de fond (7) est conformée ou fixée sur l'entretoise de soutien de pied (3) et s'étend dans, sur ou sous la plaque repose-pied (2).

5. Orthèse selon l'une des revendications précédentes,
**caractérisée en ce que** l'éclisse de bas de jambe (4) est montée sur l'entretoise de soutien de pied (3) de manière à pouvoir pivoter par rapport à l'entretoise de soutien de pied (3) autour d'un axe d'articulation (51) par l'intermédiaire d'une articulation (5).

6. Orthèse selon l'une des revendications précédentes,
**caractérisée en ce que** l'entretoise de soutien pied (3) et la plaque repose-pied (2) sont réalisées d'une seule pièce.

7. Orthèse selon l'une des revendications précédentes,
**caractérisée en ce que** la plaque repose-pied (2) et/ou l'entretoise de soutien de pied (3) sont au moins partiellement pourvues d'un enrobage (82, 83).

8. Orthèse selon l'une des revendications précédentes,
**caractérisée en ce que** l'affaiblissement (10) est réalisé en continu, sous forme de ligne discontinue ou de perforation.

9. Orthèse selon l'une des revendications précédentes,
**caractérisée en ce que** l'affaiblissement (10) s'étend sur toute la largeur de la plaque repose-pied (2).

10. Orthèse selon l'une des revendications précédentes,
**caractérisée en ce que** l'affaiblissement (10) suit le contour du bord avant (25).

11. Orthèse selon l'une des revendications précédentes,
**caractérisée en ce que** l'affaiblissement (10) est situé en direction postérieure par rapport à une articulation métatarsienne.

12. Orthèse selon l'une des revendications précédentes,
**caractérisée en ce que** plusieurs affaiblissements (10) sont disposés les uns derrière les autres à des distances régulières.

13. Orthèse selon l'une des revendications précédentes,
**caractérisée en ce que** l'affaiblissement (10) est réalisé sous forme de fente, d'évidement et/ou de réduction de matériau.

14. Orthèse selon l'une des revendications précédentes,
**caractérisée en ce qu'**un matériau (20) est disposé ou peut être disposé dans l'affaiblissement (10), qui remplit l'affaiblissement (10).

15. Orthèse selon l'une des revendications précédentes,
**caractérisée en ce que** la plaque repose-pied (2) s'étend vers l'avant non pas au-delà de l'articulation métatarso-phalangienne.
